# EUROPEAN PATENT SPECIFICATION

(11) **EP 3 579 288 B1**
(45) Date of publication and mention of the grant of the patent: **21.04.2021**
(21) Application number: 18175682.6
(22) Date of filing: 04.06.2018
(51) Int. Cl.: H01L 35/32, A61B 5/00

(54) **THERMOELECTRIC ENERGY GENERATOR ARRANGEMENT**
THERMOELEKTRISCHE ENERGIEGENERATORANORDNUNG
DISPOSITIF GÉNÉRATEUR D'ÉNERGIE THERMOÉLECTRIQUE

(43) Date of publication of application: 11.12.2019
(73) Proprietor: Polar Electro Oy, 90440 Kempele (FI)
(72) Inventor: Rytky, Pekka, 90440 Kempele (FI)
(74) Representative: Kolster Oy Ab

(56) References cited:
- WO-A1-2017/212120
- US-A1- 2004 093 041
- US-A1- 2013 087 180
- US-A1- 2017 365 766

## Description

### TECHNICAL FIELD

The present invention relates to a field of thermoelectric energy generators that convert heat flux into electric energy.

### TECHNICAL BACKGROUND

A thermoelectric generator (TEG) is a solid state device that employs temperature gradient in harvesting electric energy. A typical use case has been to employ a TEG in converting waste heat into electrical energy to charge a battery or for another purpose. Design of the TEG configuration is crucial in the sense that the heat flux tends to reduce the temperature gradient which causes reduction in efficiency of the TEG.

US 2017/0365766 relates to wearable electronic devices with thermoelectric devices. The wearable electronic device may comprise a user interface for displaying information to a user. The thermoelectric device may comprise a heat collecting unit, a thermoelectric element, and a heat expelling unit. During use, the thermoelectric element may generate power upon the flow of thermal energy from the heat collecting unit, across the thermoelectric element, and to the heat expelling unit.

### BRIEF DESCRIPTION

The present invention is defined by the subject matter of the independent claim.

Embodiments are defined in the dependent claims.

### BRIEF DESCRIPTION OF THE DRAWINGS

In the following the invention will be described in greater detail by means of preferred embodiments with reference to an accompanying drawings in which:
Figure 1 illustrates a sensor arrangement comprising a TEG element in accordance with some embodiments of the invention;
Figure 2 illustrates the TEG element according to an embodiment in a situation where the operation is reversed;
Figure 3 illustrates another embodiment of the sensor arrangement comprising the TEG element; and
Figure 4 illustrates some implementations of the sensor arrangement.

### DETAILED DESCRIPTION OF THE INVENTION

The following embodiments are exemplifying. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

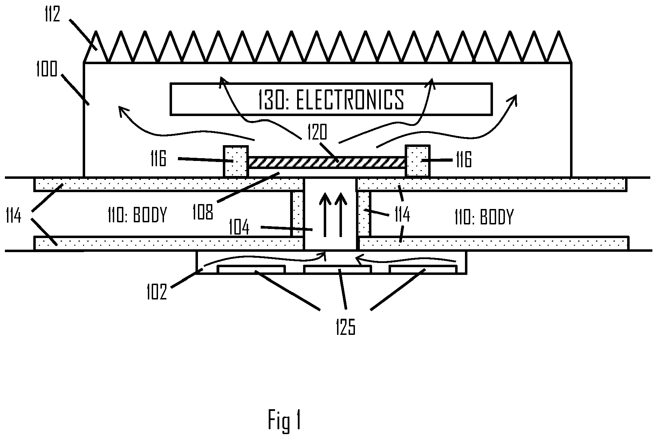

Figure 1 illustrates as sensor structure according to an embodiment of the invention. The sensor structure comprises a housing 100 arranged to be attached to a body 110. The body may comprise flexible material suitable for attaching the sensor structure to a user's body, e.g. textile or flexible plastics. The body may be a garment such as a shirt, a hat or a head gear, a swimming suit, a harness, or a strap, for example. The strap may be a head strap, a wrist strap, or a chest strap.

The sensor structure further comprises a thermoelectric generator (TEG) element 120 comprised in the housing 100 and configured to convert temperature gradient into electric energy. The sensor structure further comprises a heat collecting surface 102 coupled to the housing 100 on a side arranged to face a skin when the body is attached to the user, e.g. worn by a user. A heat conduction channel 104 is coupled between the heat collecting surface 102 and the TEG element 120, and the heat conduction channel is dimensioned such that a cross-sectional area of the heat conduction channel is smaller than the heat collecting surface 102, as illustrated in Figure 1. The heat conducting channel 104 conveys the heat to a hot plate 108 that is arranged into contact with the TEG element. The hot plate may comprise thermally conductive material, and it is coupled between the heat conducting channel and the TEG element and coupled directly to both of them. The area of the hot plate is larger than the cross-sectional area of the heat conducting channel 104.

A heat sink 112 is coupled to the housing on a side arranged to face away from the skin when the body is coupled with the user, e.g. the garment is worn by the user. The heat sink is coupled to the TEG element such that the heat sink conducts heat away from a cold side of the TEG element and dissipates the heat into air.

The housing 100 is attached to the body 110 such that the body surrounds the heat conducting channel 104 and provides thermal insulation between the heat collecting surface 102 and the heat sink 112. The heat conducting channel 104 may be arranged through a hole in the body, and the heat collecting surface 102 may have a larger diameter or area than a diameter or area of the hole in the body.

As illustrated in Figure 1, the body has a dual purpose: it serves to attach the sensor structure to the user's skin and it functions as the thermal insulator for the TEG element. The housing 100 is provided on the side of the body away from the user's skin such that maximal heat flux is allowed for the cold side of the TEG element 120. The TEG element 120 may also be provided on the same side, and only the heat collecting surface and a part of the heat conducting channel 104 are provided on the same side of the body 110 as the skin.

Heat flux is illustrated in Figure 1 by arrows. The heat collecting surface 102 funnels the heat to the heat conducting channel 104 configured to deliver the heat to a hot side of the TEG element. A cold side of the TEG element, on the other hand, is thermally coupled to the heat sink 112 to provide efficient heat flux and heat dissipation. The heat sink 112 may comprise a heat dissipation surface (serrated part illustrated in Figure 1) arranged to dissipate heat to air and further comprises thermal conduction element arranged to conduct heat from the cold side of the TEG element to the heat dissipation surface. The thermal conduction element may be formed of the housing and comprise thermally conductive material such as metal, thermally conductive silicone, thermally conductive adhesive such as epoxy, or thermally conductive plastic. In another embodiment, the thermal conduction element may be a heat pipe or another component comprised in the housing and funnelling the heat from the TEG element to the heat dissipation surface. The thermal conduction element may be thermally isolated from the hot side of the TEG element by least one insulation element 116 coupled to the hot side. The at least one insulation element 116 may also prevent heat flux to the cold side from anywhere else but from the hot side. This arrangement improves the efficiency of the TEG element by improving the thermal gradient at the TEG element.

In an embodiment, at least one insulation layer 114 is provided between the heat collecting surface 102 and the heat sink 112 and surrounding the heat conducting channel 104. As illustrated in Figure 1, the thermal insulation layer 114 may be arranged between the housing 100 and the body 114 to prevent heat flux to the TEG element from the cold side. The thermal insulation layer 114 may be arranged between the heat collecting surface 102 and the body 114 to funnel the heat from the heat collecting surface to the heat conducting channel. The thermal insulation layer 114 may surround the heat conducting channel 104 to thermally isolate the heat conducting channel and improve heat flux from the heat collecting surface 102 to the hot side of the TEG element. In all embodiments, the body serves as a further thermal insulation layer. The further insulation layer 114, however, improves the thermal insulation capabilities in certain situations, e.g. when the garment gets wet during a physical exercise. The insulation layer 114 also prevents direct contact between the body 110 and the heat collecting surface 102 and direct contact between the body 110 and the heat sink 112. The material of the insulation layer may be thermally insulating silicone foam or aerogel, for example.

Since the housing comprising the TEG element is built mainly on the side of the body facing the air, the heat dissipation may be improved and the delivery of the heat flux to the hot side of the TEG element may be controlled through the heat conducting channel by using the body as the further insulation layer. The dimensions of the heat collecting surface, heat conducting channel, and the insulation layer 114 may be designed to provide improved directed heat flux from the heat collecting surface to the hot side of the TEG element. This will allow efficient heat flux from the cold side to the heat sink 112 such that the thermal gradient at the TEG element may be improved.

Skin temperature is typically about 34 degrees Celsius when the user is at rest, while the temperature at the heat sink 112 may be room temperature or slightly higher because of the heat flux, at around 25 degrees Celsius. The achievable temperature gradient for the TEG element may be between 4 and 9 degrees Celsius which would provide approximately 320 to 720 micro Watts (uW) power per square centimetre (cm²). Such power is sufficient for low-power sensors.

The housing 100 may be attached to the body 110 with a snap-on connector. For example, the housing may comprise one counterpart of the snap-on connector, and the heat collecting surface 102 may be comprised in another counterpart of the snap-on connector. The interface of the snap-on connector may be provided at an end or in the heat conducting channel 104, for example.

The material of the heat collecting surface and the heat conducting channel should be made of material capable of efficiently transferring heat to the TEG element. The material of the heat collecting surface may be thermally conductive metal, plastic or adhesive, for example. The material of the heat conducting channel may be thermally conductive metal, plastic or adhesive, for example.

In an embodiment, the electric signal acquired by using the TEG element 120 is used to charge a battery or another energy storage comprised in the housing. The stored energy may be used for electric functions of the sensor arrangement. Let us describe such functions next.

In an embodiment, the sensor arrangement comprises a sensor head 125 on the heat collecting surface 102, a measurement circuitry 130 configured to process a measurement signal acquired by using the sensor head 125, and a signal line (not shown) coupling the sensor head with the measurement circuitry through the body 110. The signal line or lines may travel through the same hole as the heat conducting channel 104, or a separate route may be provided for the signal line(s). Depending on the capabilities of the sensor arrangement, the measurement circuitry 130 may perform various signal processing functions. The measurement circuitry may, for example, comprise a differential amplifier for an electrocardiogram (ECG) sensor head, an optical signal processing circuitry for a photoplethysmogram (PPG) sensor head, etc. In an embodiment, at least a part of the measurement circuitry may be comprised in the sensor head 125, e.g. the sensor head coupled to the heat collecting surface may comprise one or more integrated circuits of the measurement circuitry, e.g. the differential amplifier.

The housing 100 may further house a wireless communication circuitry providing the sensor arrangement wireless communication capability. The wireless communication circuitry may be configured to support one or more wireless communication protocols such as Bluetooth®, Bluetooth Low Energy, or ANT. The wireless communication circuitry may be configured to wirelessly transmit measurement data processed by the measurement circuitry.

In an embodiment, the material of the heat collecting surface is arranged to have electrically conductive properties in addition to the thermal conduction capabilities. In such embodiments, the heat collecting surface may be configured to function as an electrode of an electric sensor head 125, e.g. an ECG and/or bioimpedance electrode.

In an embodiment, the measurement circuitry is configured to perform at least two different types of measurements by using the heat collecting surface as the electrode of the sensor head. The at least two different types of measurements may comprise at least two of the following measurements: electrocardiogram measurement, galvanic skin response measurement, and bioimpedance measurement. In an embodiment, the heat collecting surface comprises two or more separate surfaces electrically isolated from one another. Then, one surface may function as a first electrode while the other surface(s) function as further electrode(s). The surfaces may be electrically isolated but thermally connected to each other by suitable selection of materials on the heat collecting surface. In another embodiment, the heat collecting surface may comprises only one electrode but the measurement circuitry may comprise a switching circuitry configured to switch the measurement function of the electrode. For example, the electrode may be used for ECG and bioimpedance measurements, and the switching circuitry may connect the electrode to different circuits of the measurement circuitry, depending on the selection of the measurement function.

In an embodiment where the sensor arrangement comprises the ECG sensor head and the PPG sensor head, both sensor heads may be comprised in the heat collecting surface. The measurement circuitry may then be configured to receive and process measurement signals received from the ECG sensor head and the PPG sensor head and to compute a pulse transit time (PTT) of a heart pulse from the measurement signals. The PTT correlates with velocity of the heart pulse between two measurement locations. A timing when the heart pulse is measured by the ECG sensor represents the timing of the heart pulse at the heart. The timing of the heart pulse detected by the PPG sensor represents the timing of the heart pulse at the measurement location of the PPG sensor head. The time difference between the two timings represents the PTT between the two locations. Blood pressure, for example, may be computed from the PTT by the measurement circuitry by using the sensor arrangement described herein.

In an embodiment, the sensor arrangement comprises a further electrode arranged on the same side of the body as the heat sink, and a signal line may connect the further electrode to the measurement circuitry. In such an embodiment, the electrode in the sensor head of the heat collecting surface may function as a ground for the ECG measurement, and the further electrode may be the measurement electrode, or vice versa. The user may perform instant measurement of the heart rate, for example, by bringing a finger on the further electrode. For the purpose of continuous heart rate measurement, the measurement circuitry may be coupled to a further electrode contacting the user's skin on the same side of the body as the heat collecting surface. The further electrode may in this case be provided at a suitable distance from the sensor head 125 to provide sufficient grounding. A signal line may lead from the further electrode to the measurement circuitry through the body 110. The signal line may be integrated into the body.

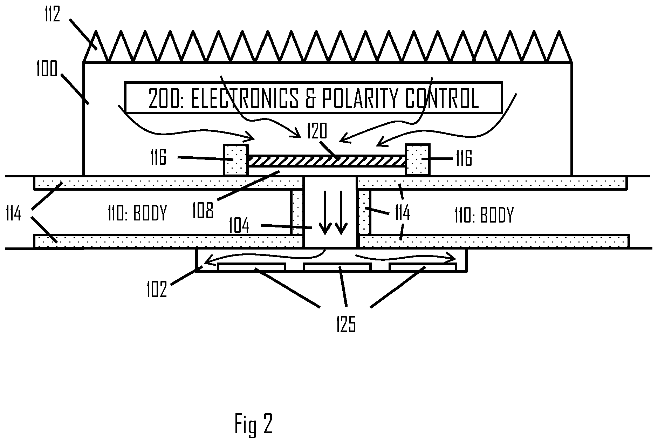

In the embodiments described above, the heat collecting surface faces the skin and the heat sink is provided on the side of the body that faces the air. In another embodiment, the hot side and the cold side is flipped. Figure 2 illustrates a solution where the operation of the arrangement for the TEG element is switched. Such a situation may occur in extreme hot climates where the air is warmer than the skin temperature. In such a case, the roles of the heat sink and the heat collecting surface are reversed, and also the heat flux will reverse, as illustrated in Figure 2. The heat sink of Figure 1 facing the air now operates as the heat collecting surface while the heat collecting surface of Figure 1 now operates as the heat sink. The heat conducting channel would deliver the heat from the TEG element towards the user's skin. The thermal conduction element described above, e.g. the heat pipe, would then deliver the heat from the surface of the heat pipe or housing to the TEG element. Such a reversal may be spontaneous operation of the arrangement, and it would cause the polarity of the electric signal output by the TEG element to switch. To exploit such a change, a polarity control circuitry 200 may be provided in the housing to electronically switch the polarity of the electric signal output by the TEG element. The polarity control circuitry may comprise a regulator that is arranged to regulate the polarity of the electric signal output by the TEG element 120.

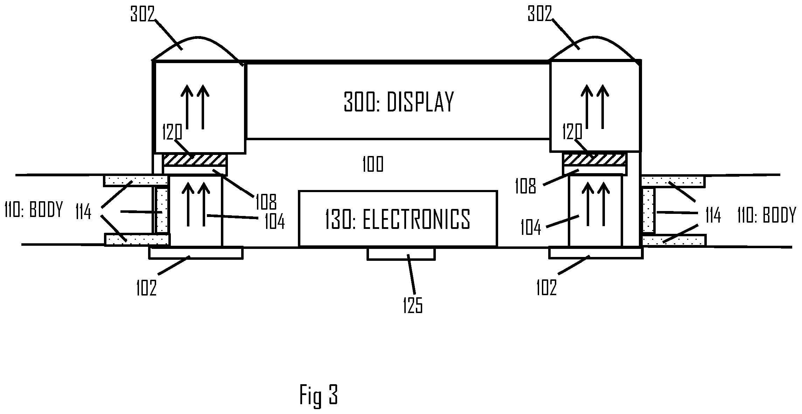

Figure 3 illustrates yet another embodiment of the sensor structure. In the embodiment of Figure 3, the TEG element is provided as an annular or semi annular element formed on an outer rim of a housing 100 of a wrist device or another wearable electronic device comprising at least one sensor. The elements of Figure 3 denoted by the same reference numbers as in Figures 1 and 2 represent the same or substantially similar elements. In the embodiment of Figure 3, a bezel 302 surrounding a display 300 of the device operates as the heat sink for the TEG element 120. The bezel may be directly coupled to the cold side of the TEG element 120 or through the above-described thermal conduction element between the bezel and the TEG element. In this embodiment, the housing 100 may extend to such that the housing at least partially surrounds sides of the heat conducting channel 104. The heat conducting channel 104 may also have an annular form, as well as the heat collecting surface(s) 102. In this embodiment, the sensor head 125, e.g. the PPG sensor head, may be comprised or coupled to the surface of the housing 100 that faces the skin. However, the heat collecting surface(s) may still form one or more sensor heads, e.g. ECG or bioimpedance electrode(s), according to the teachings described above.

The insulation layers 114 may be formed between the body 114 and the heat conducting channel 104. The body may be a wrist strap in this embodiment. However, the insulation layers 114 may also be omitted in other embodiments.

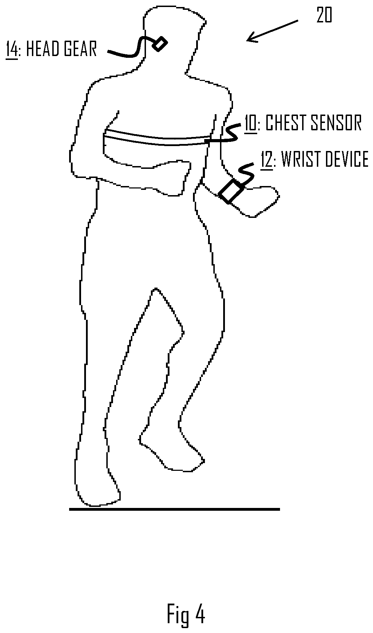

Figure 4 illustrates some applications of the sensor structure described herein. The sensor structure may be comprised in head gear 14 worn by the user 20. The head gear may comprise a band, a head strap, head dress, an ear piece, or goggles, for example. The sensor structure may form a chest sensor 10 attachable or attached to a chest strap, shirt, or swimming suit, for example. In the embodiments where the body 110 is a garment or an apparel such as the shirt or a body suit, the sensor structure may be provided on a chest, shoulder, neck, or backside area of the garment or the apparel. The sensor structure may be comprised in a wrist device 12, e.g. according to the embodiment of Figure 1 or 3.

The embodiments described above are suitable for a sensor arrangement for use in physical training, for example, when the sensor arrangement is worn by the user. The housing is small enough that it can be worn without effort and coupled to the body such that it will sustain during intensive exercises. The housing may be made waterproof. The invention and its embodiments are not limited to the examples described above but may vary within the scope of the claims.

## Claims

1. A sensor structure, comprising:
a housing (100) arranged to be attached to a body (110);
a thermoelectric generator element (120) comprised in the housing and configured to convert temperature gradient into electric energy;
a hot plate (108) arranged into contact with the thermoelectric generator element;
a heat collecting surface (102) coupled to the housing on a side arranged to face a skin when the body is coupled to a user of the sensor structure;
a heat conduction channel (104) coupled between the heat collecting surface and the hot plate, wherein a cross-sectional area of the heat conduction channel is smaller than the heat collecting surface and smaller than an area of the hot plate; and
a heat sink (112) coupled to the housing on a side arranged to face away from the skin when the body is coupled to the user.

2. The sensor structure of claim 1, further comprising:
a sensor head (125) on the heat collecting surface;
a measurement circuitry (130) configured to process a measurement signal acquired by using the sensor head; and
a signal line coupling the sensor head with the measurement circuitry through the body.

3. The sensor structure of claim 2, wherein the heat collecting surface forms an electrode of the sensor head.

4. The sensor structure of claim 3, wherein the measurement circuitry is configured to perform at least two different types of measurements by using the heat collecting surface as the electrode of the sensor head.

5. The sensor structure of claim 4, wherein the at least two different types of measurements comprise at least two of the following measurements, electrocardiogram measurement, galvanic skin response measurement, and bioimpedance measurement.

6. The sensor structure of any preceding claim 2 to 5, wherein the sensor head comprises a photoplethysmogram sensor head.

7. The sensor structure of claim 6, further comprising an electrocardiogram sensor, wherein the measurement circuitry is configured to receive and process measurement signals received from the electrocardiogram sensor and the photoplethysmogram sensor and to compute a pulse transit time of a heart pulse from the measurement signals.

8. The sensor structure of any preceding claim 2 to 7, further comprising an electrode arranged on the same side of the body as the heat sink, and a signal line connecting the electrode to the measurement circuitry.

9. The sensor structure of any preceding claim, wherein the thermoelectric generator element has an annular or semi annular shape, and the heat sink is comprised in an annular bezel (302) of a wrist device.

10. The sensor structure of any preceding claim, further comprising at least one insulation layer (114) between the heat collecting surface and the heat sink and surrounding the heat conducting channel.

11. The sensor structure of any preceding claim, further comprising means (200) for reversing polarity of an electric signal output by the thermoelectric generator element when the polarity of the electric signal switches as a result of reversed operation of the thermoelectric generator element.

12. The sensor structure of any preceding claim, further comprising at least one insulation element (116) thermally isolating a hot side of the thermoelectric generator element from the heat sink.

13. The sensor structure of any preceding claim, wherein the heat sink comprises a heat dissipation surface arranged to dissipate heat to air and further comprises thermal conduction element arranged to conduct heat from a cold side of the thermoelectric generator component to the heat dissipation surface.

14. The sensor structure of any preceding claim, wherein the housing arranged to be attached to a shirt, hat, swimming suit, harness, or a wrist strap.

15. The sensor structure of any preceding claim, wherein when the housing is attached to the body, the body surrounds the heat conducting channel and provides thermal insulation between the heat collecting surface and the heat sink.

## Patentansprüche

1. Eine Sensorstruktur, die umfasst:
einem Gehäuse (100), das so angeordnet ist, dass es an einem Körper (110) angebracht ist;
einem thermoelektrischen Generatorelement (120), das im Gehäuse untergebracht ist und so konfiguriert ist, dass der Temperaturgradient in elektrische Energie umgewandelt wird;
einer Heizplatte (108), die in Kontakt mit dem thermoelektrischen Generatorelement angeordnet ist;
einer Wärmeaufnahmefläche (102), die an das Gehäuse auf einer Seite gekoppelt ist, die einer Haut zugewandt angeordnet ist, wenn der Körper an einen Benutzer der Sensorstruktur gekoppelt ist;
einem Wärmeleitungskanal (104), der zwischen der Wärmeaufnahmefläche und der Heizplatte gekoppelt ist, wobei ein Querschnittsbereich des Wärmeleitungskanals kleiner als die Wärmeaufnahmefläche und kleiner als ein Bereich der Heizplatte ist; und
einem Kühlkörper (112), der an das Gehäuse an einer Seite gekoppelt ist, die von der Haut abgewandt angeordnet ist, wenn der Körper an den Benutzer gekoppelt ist.

2. Die Sensorstruktur nach Anspruch 1, die ferner umfasst:
einen Sensorkopf (125) auf der Wärmeaufnahmefläche;
eine Messschaltung (130), die so konfiguriert ist, dass ein Messsignal verarbeitet wird, das unter Einsatz des Sensorkopfs erfasst wird; und
eine Signalleitung, die den Sensorkopf an die Messschaltung über den Körper koppelt.

3. Die Sensorstruktur nach Anspruch 2, wobei die Wärmeaufnahmefläche eine Elektrode des Sensorkopfs bildet.

4. Die Sensorstruktur nach Anspruch 3, wobei die Messschaltung so konfiguriert ist, dass mindestens zwei verschiedene Arten von Messungen unter Einsatz der Wärmeaufnahmefläche als Elektrode des Sensorkopfs durchgeführt werden.

5. Die Sensorstruktur nach Anspruch 4, wobei die mindestens zwei verschiedenen Arten von Messungen mindestens zwei der folgenden Messungen, Elektrokardiogrammmessung, galvanische Hautreaktionsmessung und Bioimpedanzmessung, umfassen.

6. Die Sensorstruktur nach einem der vorstehenden Ansprüche 2 bis 5, wobei der Sensorkopf einen Photoplethysmogrammsensorkopf umfasst.

7. Die Sensorstruktur nach Anspruch 6, die ferner einen Elektrokardiogrammsensor umfasst, wobei die Messschaltung so konfiguriert ist, dass Messsignale empfangen und verarbeitet werden, die vom Elektrokardiogrammsensor und vom Photoplethysmogrammsensor eingehen, und dass eine Pulsübergangszeit des Herzimpulses aus den Messsignalen berechnet wird.

8. Die Sensorstruktur nach einem der vorstehenden Ansprüche 2 bis 7, die ferner eine Elektrode, die an derselben Seite des Körpers wie der Kühlkörper angeordnet ist, und eine Signalleitung, die die Elektrode mit der Messschaltung verbindet, umfasst.

9. Die Sensorstruktur nach einem der vorstehenden Ansprüche, wobei das thermoelektrische Generatorelement eine ringförmige oder halbringförmige Form aufweist und der Kühlkörper in eine ringförmige Blende (302) eines Handgelenksgeräts integriert ist.

10. Die Sensorstruktur nach einem der vorstehenden Ansprüche, die ferner mindestens eine Isolierschicht (114) zwischen der Wärmeaufnahmefläche und dem Kühlkörper umfasst und umlaufend um den Wärmeleitungskanal angeordnet ist.

11. Die Sensorstruktur nach einem der vorstehenden Ansprüche, die ferner Mittel (200) zum Umkehren der Polarität einer elektrischen Signalausgabe durch das thermoelektrische Generatorelement umfasst, wenn die Polarität des elektrischen Signals als Ergebnis eines Reversierbetriebs des thermoelektrischen Generatorelements umschaltet.

12. Die Sensorstruktur nach einem der vorstehenden Ansprüche, die ferner mindestens ein Isolierelement (116) umfasst, das eine warme Seite des thermoelektrischen Generatorelements vom Kühlkörper thermisch isoliert.

13. Die Sensorstruktur nach einem der vorstehenden Ansprüche, wobei der Kühlkörper eine Wärmeableitfläche umfasst, die so angeordnet ist, dass Wärme an die Luft abgeleitet wird, und ferner ein wärmeleitendes Element umfasst, das so angeordnet ist, dass Wärme von einer kalten Seite der thermoelektrischen Generatorkomponente zur Wärmeableitfläche geleitet wird.

14. Die Sensorstruktur nach einem der vorstehenden Ansprüche, wobei das Gehäuse so angeordnet ist, dass es an einem T-Shirt, einer Kopfbedeckung, einem Badeanzug, einem Gurt oder einem Armband befestigt wird.

15. Die Sensorstruktur nach einem der vorstehenden Ansprüche, wobei, wenn das Gehäuse am Körper angebracht ist, der Körper den Wärmeleitungskanal umgibt und eine Wärmeisolierung zwischen der Wärmeaufnahmefläche und dem Kühlkörper gewährleistet.

## Revendications

1. Structure de capteur, qui comprend :
un boîtier (100) prévu pour être fixé sur un corps (110) ;
un élément de générateur thermoélectrique (120) compris dans le boîtier et configuré pour convertir un gradient de température en énergie électrique ;
une plaque chauffante (108) prévue en contact avec l'élément de générateur thermoélectrique ;
une surface de collecte de chaleur (102) couplée au boîtier sur un côté prévu pour faire face à une peau lorsque le corps est couplé à un utilisateur de la structure de capteur ;
un canal de conduction de chaleur (104) couplé entre la surface de collecte de chaleur et la plaque chauffante, dans lequel une surface transversale du canal de conducteur de chaleur est plus petite que la surface de collecte de chaleur et plus petite qu'une surface de la plaque chauffante ; et
un dissipateur thermique (112) couplé au boîtier sur un côté prévu pour ne pas faire face à la peau lorsque le corps est couplé à l'utilisateur.

2. Structure de capteur selon la revendication 1, qui comprend en outre :
une tête de capteur (125) sur la surface de collecte de chaleur ;
des circuits de mesure (130) configurés pour traiter un signal de mesure acquis à l'aide de la tête de capteur ; et
une ligne de signal qui couple la tête de capteur aux circuits de mesure par le biais du corps.

3. Structure de capteur selon la revendication 2, dans laquelle la surface de collecte de chaleur forme une électrode de la tête de capteur.

4. Structure de capteur selon la revendication 3, dans laquelle les circuits de mesure sont configurés pour effectuer au moins deux types différents de mesures en utilisant la surface de collecte de chaleur comme l'électrode de la tête de capteur.

5. Structure de capteur selon la revendication 4, dans laquelle les au moins deux types différents de mesures comprennent au moins deux des mesures suivantes : une mesure d'électrocardiogramme, une mesure de réflexe psychogalvanique, et une mesure de bio-impédance.

6. Structure de capteur selon l'une quelconque des revendications 2 à 5, dans lequel la tête de capteur comprend une tête de capteur de photopléthysmogramme.

7. Structure de capteur selon la revendication 6, qui comprend en outre un capteur d'électrocardiogramme, dans laquelle les circuits de mesure sont configurés pour recevoir et traiter les signaux de mesure reçus de la part du capteur d'électrocardiogramme et du capteur de photopléthysmogramme, et pour calculer un temps de transit d'impulsion d'une impulsion cardiaque à partir des signaux de mesure.

8. Structure de capteur selon l'une quelconque des revendications 2 à 7, qui comprend en outre une électrode prévue du même côté du corps que le dissipateur thermique, et une ligne de signal qui relie l'électrode aux circuits de mesure.

9. Structure de capteur selon l'une quelconque des revendications précédentes, dans laquelle l'élément de générateur thermoélectrique possède une forme annulaire ou semi-annulaire, et le dissipateur thermique est compris sur une facette annulaire (302) d'un dispositif de poignet.

10. Structure de capteur selon l'une quelconque des revendications précédentes, qui comprend en outre au moins une couche d'isolation (114) entre la surface de collecte de chaleur et le dissipateur thermique, et qui entoure le canal de conduction de chaleur.

11. Structure de capteur selon l'une quelconque des revendications précédentes, qui comprend en outre un moyen (200) destiné à inverser la polarité d'un signal électrique délivré par l'élément de générateur thermoélectrique lorsque la polarité du signal électrique change à la suite d'un fonctionnement inversé de l'élément de générateur thermoélectrique.

12. Structure de capteur selon l'une quelconque des revendications précédentes, qui comprend en outre au moins un élément d'isolation (116) qui isole thermiquement un côté chaud de l'élément de générateur thermoélectrique du dissipateur thermique.

13. Structure de capteur selon l'une quelconque des revendications précédentes, dans laquelle le dissipateur thermique comprend une surface de dissipation de chaleur prévue pour dissiper la chaleur dans l'air, et comprend en outre un élément de conduction thermique prévu pour conduire la chaleur d'un côté froid du composant de générateur thermoélectrique vers la surface de dissipation de chaleur.

14. Structure de capteur selon l'une quelconque des revendications précédentes, dans laquelle le boîtier est prévu pour être fixé sur une chemise, un chapeau, un maillot de bain, un harnais, ou une dragonne.

15. Structure de capteur selon l'une quelconque des revendications précédentes, dans laquelle, lorsque le boîtier est fixé sur le corps, le corps entoure le canal de conduction de chaleur et assure une isolation thermique entre la surface de collecte de chaleur et le dissipateur thermique.
